# EUROPEAN PATENT APPLICATION

(11) **EP 1 686 118 A1**
(43) Date of publication of application: **02.08.2006**
(21) Application number: 06250293.5
(22) Date of filing: 19.01.2006
(51) Int. Cl.: C07D 233/32, C07C 67/03, C07C 69/54

(54) **Transesterification process for production of (meth)acrylate ester monomers**

(30) Priority: 28.01.2005 US 648003 P
(71) Applicant: Rohm and Haas Company, Philadelphia, PA 19106-2399 (US)
(72) Inventor: Curtis, Michael A., Huston, texas 77005 (US)
(74) Representative: Kent, Venetia Katherine

(57) **Abstract**

This invention utilizes multiple charges of a transesterification catalyst in a transesterification process for the production of esters of alkyl (meth)acrylate monomers. The multiple catalyst charge addition strategy and reaction conditions provide for higher productivity and enhanced consistency.

## Description

### FIELD OF THE INVENTION

The present invention relates to a transesterification process for the production of (meth)acrylate ester monomers from alkyl (meth)acrylates and alcohols. More particularly, the present invention is an improved transesterification process which involves addition of a transesterification catalyst as separate, multiple charges during the period of reaction.

### BACKGROUND OF THE INVENTION

Esters of (meth)acrylates may be formed by transesterification of an alkyl (meth)acrylate with a selected alcohol, in the presence of a transesterification catalyst, followed by the elimination of a low boiling by-product alcohol in the form of an azeotrope with the alkyl (meth)acrylate. Prior dehydration of the reaction mixture is of particular advantage where the transesterification catalyst employed is susceptible to deactivation in the presence of water.

More particularly, typical transesterification processes have involved feeding all of the reactants into a reactor and dehydrating the resulting reaction mixture by azeotropic distillation. Once the residual water level is acceptable, a suitable transesterification catalyst is added to the reaction mixture, and the mixture is heated to commence the transesterification reaction. The reaction products typically include the desired (meth)acrylate ester and a low boiling by-product alcohol. Azeotropic distillation is conducted to separate and remove the alcohol by-product, along with some of the starting alkyl (meth)acrylate. The temperature of the reaction mixture rises as the alcohol by-product is removed. Because the transesterification process is reversible, the rate of reaction is largely determined by rate of removal of the by-product alcohol.

Tin-based transesterification catalysts, such as dibutyl tin oxide and dibutyl tin dimethoxide, have been used in transesterification processes to produce esters of (meth)acrylates. For example, U.S. 5,210,199 discloses a transesterification process for producing esters from methyl methacrylate using dibutyl tin oxide as the catalyst. More particularly, a methyl methacrylate and alcohol mixture was dehydrated, and then a dibutyl tin catalyst was added to the mixture, all at once in a single charge. The disclosure of U.S. 5,210,199 recommends that the maximum dehydration be reached before adding the catalyst, without providing a particular or preferred water content.

Due to environmental concerns, tin-based catalysts are being replaced by other, more environmentally friendly catalysts, such as, for example, alkali metals and alkali metal hydroxides, such as lithium and lithium hydroxide. For example, a transesterification process for production of (meth)acrylate esters is disclosed in U.S. 5,072,027, wherein the catalyst of choice was anhydrous lithium hydroxide obtained from drying lithium hydroxide monohydrate under certain conditions. The lithium catalyst was added, all at once in a single charge, to a mixture of methyl methacrylate and alcohol after the mixture was dehydrated to 1000 ppm or less, based on the total weight of the mixture.

Another transesterification process which uses a lithium hydroxide monohydrate catalyst is disclosed in U.S. 6,515,138 and also involves the one-time addition of catalyst, in a single charge, to a reaction mixture which contains methyl methacrylate and alcohol and which has been dehydrated.

U.S. 4,791,221 discloses a process for transesterification of methyl methacrylate with alcohol in the presence of a lithium-based catalyst and an azeotrope-forming compound. The lithium-based catalyst was added incrementally, or gradually, to the reaction mixture, over a period of from one tenth to one half of the overall period of reaction, with the goal of minimizing the catalyst concentration in the reaction mixture, thereby preventing unwanted reaction of the catalyst with the reagents. Such gradual introduction of the catalyst was achieved by dissolving the catalyst in a carrier solvent, such as methanol, and continuously feeding the catalyst-containing solution to the reaction mixture. The purpose of the azeotrope-forming compound is to produce an azeotrope with methanol that boils at a temperature of less than about 60°C, thereby allowing removal of the methanol from the reaction mixture and avoiding formation of a methyl methacrylate/methanol azeotrope. There is no disclosure or suggestion, however, in U.S. 4,791,221, of adding the catalyst to the reaction mixture as multiple, separate catalyst charges, or shots, nor of the benefits achieved thereby.

### SUMMARY OF THE INVENTION

It has been surprisingly discovered that the one-time addition of a single charge of transesterification catalyst, as traditionally practiced in connection with transesterification processes, results in insufficient (meth)acrylate ester product yields unless very strict conditions (e.g., low water content and high reactant (meth)acrylate-to-alcohol ratio) are maintained, which results in limited productivity. It has also surprisingly been found that addition of multiple, separate charges of the catalyst to the reaction mixture, over the course of the reaction, provides greater reactor efficiency and/or productivity than achieved by other known methods, and also requires less stringent conditions (for example, the reaction mixture may be dehydrated to a lesser degree) than previous processes. Addition of the catalyst in the form of multiple, separate charges has now been found to provide improved reactor efficiency even as compared to processes wherein the catalyst is gradually, continuously introduced to the reaction mixture.

The present invention provides a transesterification process for the production of a (meth)acrylate ester, comprising the steps of:
(a) forming a reaction mixture, comprising:
   (1) water and at least one alkyl (meth)acrylate having Formula I: 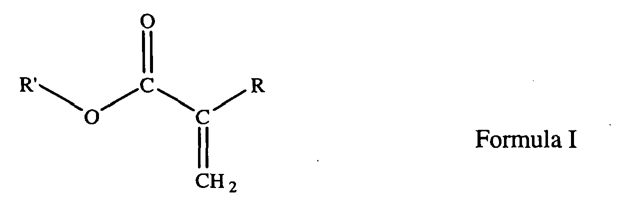 wherein R = H or CH₃; and
      wherein R' = C₁-C₈ straight or branched alkyl;
   (2) at least one alcohol selected from the group consisting of aliphatic linear or branched chain monoalcohols, cycloaliphatic alcohols, aromatic alcohols, functional alcohols, unsaturated alcohols, analiphatic polyols, alcohols of ethylene oxide adduct of ethylene urea, and mixtures thereof; and
   (3) 10 to 10,000 ppm, based on the weight of said at least one alcohol, of at least one polymerization inhibitor;
(b) removing water from said reaction mixture by azeotropic distillation until said reaction mixture has a water content of no more than 1200 ppm based on the total weight of said reaction mixture;
(c) adding to said reaction mixture at least two charges of a transesterification catalyst, wherein each charge comprises 0.1 to 10 mole % of said transesterification catalyst, based on the total moles of said at least one alcohol,
   said transesterification catalyst being selected from the group consisting of dibutyl tin oxide, reaction products of dibutyl tin oxide with components in the transesterification of various alcohols with alkyl (meth)acrylates; dibutyl tin dimethoxide, reaction products of dibutyl tin dimethoxide with components in the transesterification of various alcohols with alkyl (meth)acrylates; methanolic magnesium methylate; lithium, lithium carbonate, anhydrous alkali metal hydroxide, hydrates of alkali metal hydroxide, and mixtures thereof; and
(d) when said reaction mixture has a temperature of less than about 60°C, heating said reaction mixture to at least 60°C after addition of a first of said at least two charges of said transesterification catalyst to commence reaction of said alkyl (meth)acrylate with said at least one alcohol to form a (meth)acrylate ester having Formula II: 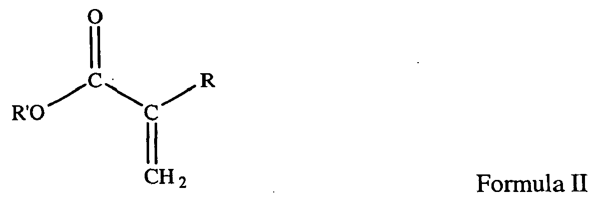 wherein R = H or CH₃, and R' O = an alkoxide of the at least one alcohol, and
   a product alcohol having Formula III:

   R'―OH Formula III

   wherein R' = C₁-C₈ straight or branched alkyl.

In a particular embodiment of the process of the present invention, the heating step is commenced within about 10 minutes after addition of the first of said at least two charges of said transesterification catalyst. Additionally, the molar ratio of the alcohol to the alkyl (meth)acrylate may be from 1:2 to 1:6.5.

In another particular embodiment of the process of the present invention, the alcohol comprises an hydroxyl alkyl imidazolidin-2-one, for example, hydroxyethyl ethylene urea, and the alkyl (meth)acrylate comprises methyl methacrylate. Moreover, the transesterification catalyst is lithium hydroxide monohydrate.

In still another embodiment of the process of the present invention, steps of (a) forming a reaction mixture and (b) removing water from said reaction mixture are accomplished by:
(A) forming a reaction mixture, comprising:
   (i) water and at least one alkyl (meth)acrylate having Formula I: 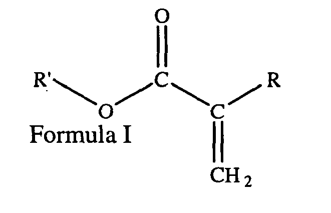 where R = H or CH₃; and
      where R' = C₁-C₈ straight or branched alkyl; and
   (ii) 10 to 10,000 ppm, based on the total weight of alcohol to be added, of at least one polymerization inhibitor selected from the group consisting of oxygen, diethylhydroxylamine, p-methoxy phenol, hydroquinone, phenothiazine, 2,6-dit-butylpara-cresol, 3,5-di-t-butyl-4-hydroxyanisole, 2,5-di-t-butylhydroxyanisole, 4-hydroxy-2,2,6,6-tetramethyl piperidinyl free radical (4-hydroxy-TEMPO), 4-methacryloyloxy-2,6,6-tetrmethyl piperidinyl free radical, and 4-hydroxy-2,6,6-tetramethyl N-hydroxy piperidine and mixtures thereof;
(B) removing water from said reaction mixture by azeotropic distillation until said reaction mixture has a water content of no more than 1200 ppm based on the total weight of said reaction mixture;
(C) adding to said reaction mixture at least one alcohol selected from the group consisting of aliphatic linear or branched chain monoalcohols, cycloaliphatic alcohols, aromatic alcohols, alcohols bearing other functional groups, alcohols of ethylene oxide adduct of ethylene urea, and mixtures thereof.

### DETAILED DESCRIPTION OF THE INVENTION

The transesterification process of the present invention produces a (meth)acrylate ester product and involves a first step of forming a reaction mixture which comprises at least one alkyl (meth)acrylate and at least one alcohol, along with a polymerization inhibitor to prevent unwanted polymerization of any meth(acrylate) containing compound.

The at least one alkyl (meth)acrylate has the following Formula I: 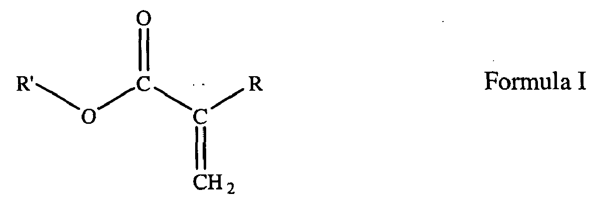 where R = H or CH₃; and where R' = C₁-C₈ straight or branched alkyl.

Several alcohols are suitable for use in the process of the present invention and include, for example, without limitation: aliphatic linear or branched chain monoalcohols, such as n-butanol, n-propanol, lauryl alcohol, stearyl alcohol, 2-ethylhexanol; cycloaliphatic alcohols, such as cyclophexanol; aromatic alcohols, such as benzyl alcohol; alcohol bearing other functional groups, such as ethylene glycol monomethylether, ethylene glycol monoisopropylether; alcohols of ethylene oxide adduct of ethylene urea, such as hydroxyethyl ethylene urea.

Suitable polymerization inhibitors include oxygen, diethylhydroxylamine, p-methoxy phenol, hydroquinone, phenothiazine, 2,6-dit-butylpara-cresol, 3,5-di-t-butyl-4-hydroxyanisole, 2,5-di-t-butylhydroxyanisole, 4-hydroxy-2,2,6,6-tetramethyl piperidinyl free radical (4-hydroxy-TEMPO), 4-methacryloyloxy-2,6,6-tetrmethyl piperidinyl free radical, and 4-hydroxy-2,6,6-tetramethyl N-hydroxy piperidine and mixtures thereof. The total amount of polymerization inhibitor added to the reaction mixture is typically from 10 to 10000 parts per million (ppm), for example, from 200 to 3000 ppm. All ranges used herein are inclusive and combinable.

Typically, the amount of alkyl (meth)acrylate reactant in the reaction mixture is in stoichiometric excess of the amount of alcohol reactant. For example, the mole ratio of alcohol to alkyl (meth)acrylate may be typically from 1:1 to 1:20, for example, without limitation, from 1:2 to 1:6.5, or even from 1:2.2 to 1:3.6. This is because, as discussed in further detail hereinafter, a product alcohol is removed, along with a portion of the alkyl (meth)acrylate reactant, from the reaction mixture by azeotropic distillation during the period of reaction. The removed mixture of alkyl (meth)acrylate and product alcohol may be further separated and the alkyl (meth)acrylate reactant may be recycled to the reaction mixture.

The process of the present invention further comprises the step of removing water from the reaction mixture. In practice, alkyl (meth)acrylates typically contain residual amounts of water or are provided in aqueous solution, to improve their viscosity characteristics and facilitate delivery to the reaction vessel. When the transesterification catalysts to be used are susceptible to deactivation in the presence of water, such as, for example, dibutyl tin oxide and lithium hydroxide catalysts, water should be removed prior to adding the catalyst to the reaction mixture. Water should be removed from the reaction mixture until the water content of the reaction mixture is no more than about 1200 ppm (i.e., about 0.12 weight %), based on the total weight of the reaction mixture.

It is possible, as described hereinabove, to combine the at least one alkyl (meth)acrylate, the at least one alcohol and the polymerization inhibitor, to form a reaction mixture and then remove water from this mixture. Alternatively, it is also possible to combine the at least one alkyl (meth)acrylate and the polymerization inhibitor, remove water therefrom, and then add the at least one alcohol thereto, thus forming the reaction mixture. The step of removing water from the reaction mixture may be accomplished, for example, without limitation, by azoetropic distillation of a mixture of water and alkyl (meth)acrylate.

A further step of the process of the present invention is adding to the reaction mixture at least two separate charges of a transesterification catalyst to the reaction mixture. Each catalyst charge comprises an amount of transesterification catalyst equal to from 0.1 to 10 mole %, based on the total moles of alcohol that are present, or which will be present, in the reaction mixture, depending upon which of the foregoing methods of forming the reaction mixture is practiced. For example, one or more of the catalyst charges may comprise transesterification catalyst in an amount of from 0.4 to 7 mole %, based on the total moles of alcohol present in the reaction mixture. The catalyst can be added by any known, conventional delivery means, such as, without limitation, via a pressurized a pressurized charge hopper, or via a parallel series of individually controlled inline chambers where the catalyst is mixed with the reaction mixture as a carrier, or into a slurry mix with, for example, methyl methacrylate.

Suitable transesterification catalysts may be selected from the group consisting of: dibutyl tin oxide, reaction products of dibutyl tin oxide with components in the transesterification of various alcohols with alkyl (meth)acrylates; dibutyl tin dimethoxide, reaction products of dibutyl tin dimethoxide with components in the transesterification of various alcohols with alkyl (meth)acrylates; methanolic magnesium methylate; lithium, lithium carbonate, anhydrous alkali metal hydroxide, hydrates of alkali metal hydroxide, and mixtures thereof.

The reaction mixture temperature, as measured at a time no more than 10 minutes preceding addition of a charge of transesterification catalyst thereto, may be from about 50°C to 120°C, for example, without limitation, from about 90°C to 100°C.

If the temperature of the reaction mixture is less than about 60°C immediately after the first charge of transesterification catalyst has been added, the reaction mixture should be heated to at least 60°C, such as, for example, to at least 90°C, in order to commence the transesterification reaction. Where such heating is necessary, the reaction mixture should be heated within about ten minutes, for example, within five minutes, or even one minute, after addition of each charge of catalyst. This heating should occur at a rate of at least 1 °C per minute, for example, at least 3°C per minute, until the target temperature is achieved.

As the transesterification reaction proceeds, the products include, but are not necessarily limited to, a product (meth)acrylate and a product alcohol that is different from the reactant alcohol or alcohols that were used to form the reaction mixture. The product (meth)acrylate ester produced by the transesterification process of the present invention has Formula II as follows: 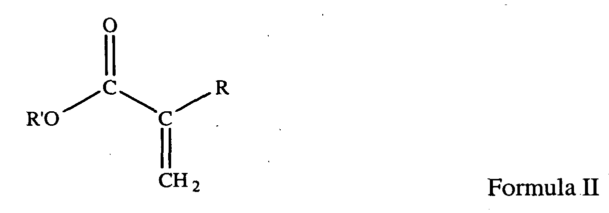 wherein R = H or CH₃, and R'O = an alkoxide of the selected reactant alcohol. The product alcohol has Formula III as follows:

R'―OH Formula III

where R' = C₁-C₈ straight or branched alkyl.

For example, when the reactant alcohol is hydroxyl alkyl imidazolidin-2-one, having the following Formula IV: 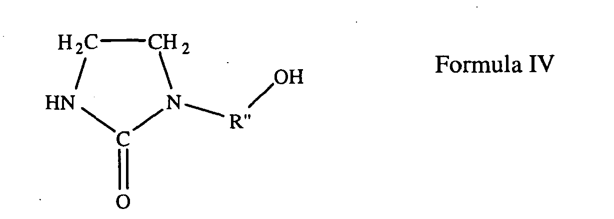 wherein R" = C₁-C₈ straight, branched or cyclic, and saturated or unsaturated, hydrocarbon, then the product (meth)acrylate ester has Formula V as follows: 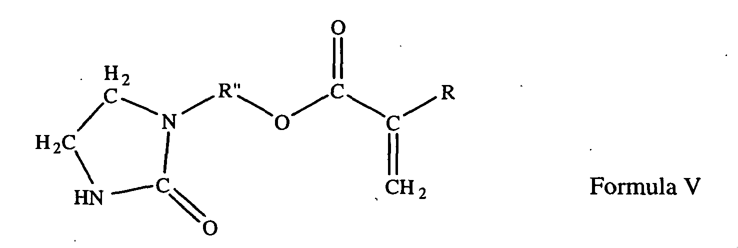 wherein R = H or CH₃, and R" = C₁-C₈ straight, branched or cyclic, and saturated or unsaturated, hydrocarbon.

The reaction temperature (i.e., the temperature of the reaction mixture during the transesterification reaction) of the process of the present invention may be from about 60°C to 140°C, for example, without limitation, 70°C, to 125°C. The reaction pressure may be from 760 mm Hg (atmospheric) to reduced or elevated pressures, such as, for example, from 400 mm Hg to 900 mm Hg.

During the course of the reaction, the product alcohol is removed from the system; by azeotropic distillation, as an azeotropic mixture of the alkyl (meth)acrylate reactant and the product alcohol.

The number and frequency of addition of subsequent catalyst charges after the first charge are determined according to when there is low production, or no production, of the by-product alcohol as the reaction proceeds. It is well within the skill of ordinary persons to determine the number and frequency of such subsequent catalyst charges. For example, the reaction temperatures, chemical analysis of the reaction mixture, and/or analysis of the collected azeotrope mixture, may be used to measure and detect the low production or absence of production of the product alcohol. Alternatively, subsequent catalyst charges may be added on a time basis, such as at predetermined time intervals, which may be evenly spaced or not. The subsequent catalyst additions are added in a similar fashion as described above where the reaction mixture is typically pre-heated to a temperature of from 70°C to 115°C prior to catalyst.

It is not necessary to remove the transesterification catalyst or catalyst by-products from the reaction mixture, especially where excess alkyl (meth)acrylate is used and/or diluted with water, or where the reaction mixture is treated with water to effect an exchange of the excess alkyl (meth)acrylate with water, via azeotropic distillation, to produce a product mixture comprising the desired ester in aqueous solution. Furthermore, even those transesterification processes which produce yields of less than 64% of certain ester products, such as N-(2-methacryloyloxyethyl) ethylene urea, may be commercially viable.

Particular embodiments of the process of the present invention will now be described in detail in connection with the following examples.

### EXAMPLES

### Comparative Example 1

This example demonstrates that a single, one-time charge of catalyst is capable of producing sufficient product yields under certain conditions, i.e., when the molar ratio of alkyl (meth)acrylate to alcohol is relatively high (i.e., 5.6 or greater), and when the dehydration step reduces the water content of the reaction mixture to a relatively low level (i.e., less than 100 ppm).

A mixture of 132.6 grams (1.02 moles) of 1-hydroxy ethylene urea (HEEU), 575.5 grams (5.75 moles) of methyl methacrylate (MMA) and 0.2 grams (0.0012 moles) of 4-hydroxy-2,2,6,6-tetramethyl piperidinyloxy, free radical (4-hydroxy TEMPO, free radical) was charged to a 1-liter 4-necked flask equipped with a temperature indicator/controller, magnetic stirrer, mixed gas (8% O₂ - 92% N₂) sparge inlet, and a 1 inch diameter-10 plate Oldershaw column fitted with a distillation head, distillate rate removal-vapor pressure temperature controller, and a graduated distillate receiver. During dehydration of the batch, the mixture was stirred, sparged with mixed gas at a rate of 70 ml/min and heated to reflux at atmospheric pressure, while removing the methyl methacrylate-water azeotrope. The maximum temperature atop the column was 99°C, and the maximum temperature in the flask was 105°C. The mixture was dehydrated to a water concentration of 72 ppm. After which, the mixture was cooled to 70°C. To the biphasic mixture was added a single catalyst charge of 0.43 grams (0.01 moles) of lithium hydroxide monohydrate. Within five minutes of adding the catalyst, heat was applied and the temperature rose at a rate of 2.3°C/min. During reaction the mixture was stirred, sparged with mix gas, and continuously heated to reflux at atmospheric pressure while removing the MMA-methanol of reaction azeotrope. Two and one-half hours later the reaction was considered complete. At this point the yield of N-(2-methacryloyloxyethyl) ethylene urea (MEEU) was 81.0%; and MEEU selectivity was 93.4%. During the reaction of the batch, the temperature at the top of the column was 45°C to 99°C and the temperature of the pot was 70°C to 107°C.

A final commercial product formulation was then prepared as follows. The mixture was cooled to 70°C and 213 grams of water was added. The flask contents were heated and the MMA-water azeotrope was removed. During the removal of excess MMA the contents were stirred and sparged. According to quantitative high-performance liquid chromatography (HPLC) the mixture contained 314 grams of product and a yellow liquid containing 43.7 weight % MEEU, 3.7 weight % HEEU, and 0.6 weight% N-(2-methacryloyloxyethyl)-N'-(methacryloyl)ethylene urea (MEMEU) and 4.4 weight % of unintentional byproducts. By Karl Fischer analysis the product contained 46.6 weight % water. A reactor efficiency ratio of 0.194 was measured by dividing pounds of MEEU produced by pounds of raw materials fed.

### Example 1

This example demonstrates that addition of multiple, separate catalyst charges to the reaction mixture provides sufficient product yields, while reducing the degree of pre-reaction dehydration that is required (i.e., dehydration to 510 ppm water content was sufficient here).

A mixture of 133.0 grams (1.02 moles) of 1-hydroxy ethylene urea (HEEU), 575.5 grams (5.75 moles) of methyl methacrylate (MMA) and 0.2 grams (0.0012 moles) of 4-hydroxy-2,2,6,6-tetramethyl piperidinyloxy, free radical (4-hydroxy TEMPO, free radical) was charged to a 1-liter 4-necked flask equipped with a temperature indicator/controller, magnetic stirrer, mixed gas (8% O₂ - 92% N₂) sparge inlet, and a 1 inch diameter-10 plate Oldershaw column fitted with a distillation head, distillate rate removal-vapor pressure temperature controller, and a graduated distillate receiver. A dehydration step was not necessary since the reaction mixture already had water content of 0.051 wt% (510 ppm). The reaction mixture was heated to 70°C and a first catalyst charge of 0.43 grams (0.01 moles) of lithium hydroxide monohydrate was added. Within five minutes of adding the catalyst, heat was applied and the temperature rose at a rate of 3°C/min. During reaction, the mixture was stirred, sparged with mix gas, and continuously heated to reflux at atmospheric pressure while removing the MMA-methanol of reaction azeotrope. After two hours of reaction, methanol generation dramatically slowed, as evident by column temperatures. The mixture was cooled to 70°C and to the single phase mixture was added a second catalyst charge of 0.43 grams (0.01 moles) of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. After one hour of reaction, methanol generation once again slowed. The mixture was cooled to 70°C and to the single phase mixture was added a third catalyst charge of 0.43 grams (0.01 moles) of lithium hydroxide monohydrate. Within five minutes of adding the catalyst, heat was applied and the temperature rose as previously stated. During reaction the mixture was stirred, sparged with mix gas, and continuously heated to reflux at atmospheric pressure while removing the MMA-methanol of reaction azeotrope. Two hours later the reaction was considered complete. At this point the yield of MEEU was 81.0%; and MEEU selectivity was 86.6%. During the reaction of the batch, the temperature at the top of the column was 20°C to 100°C and the temperature of the pot was 70°C to 115°C. A reactor efficiency ratio of 0.148 was measured by dividing pounds of MEEU produced by pounds of raw materials fed.

### Example 2

The example demonstrates that more (meth)acrylate product (greater mass) can be produced in the same size reactor, in the same time frame, using multiple catalyst charges despite the relatively low water content (i.e., 110 ppm) following pre-reaction dehydration.

A mixture of 210.4 grams (1.62 moles) of 1-hydroxy ethylene urea (HEEU), 570.0 grams (5.69 moles) of methyl methacrylate (MMA) and 0.36 grams (0.0021 moles) of 4-hydroxy-2,2,6,6-tetramethyl piperidinyloxy, free radical (4-hydroxy TEMPO, free radical) was charged to a 1-liter 4-necked flask equipped with a temperature indicator/controller, magnetic stirrer, mixed gas (8% O₂ - 92% N₂) sparge inlet, and a 1-inch diameter-10 plate Oldershaw column fitted with a distillation head, distillate rate removal-vapor pressure temperature controller, and a graduated distillate receiver. During dehydration of the batch, the mixture was stirred, sparged with mixed gas at a rate of 70 ml/min and heated to reflux at atmospheric pressure while removing the MMA-water azeotrope. The maximum temperature atop the column was 83°C, and the maximum temperature in the flask was 101°C. At the conclusion of dehydration, the mixture was cooled to 70°C. The water content at this point was 110 ppm. To the biphasic mixture was added a first catalyst charge of 0.10 grams (0.002 moles) of lithium hydroxide monohydrate. Within five minutes of adding the catalyst, heat was applied and the temperature rose at a rate of 1.3°C/min. During reaction, the mixture was stirred, sparged with mix gas, and continuously heated to reflux at atmospheric pressure while removing the MMA-methanol of reaction azeotrope. After one hour of reaction, methanol generation dramatically slowed, as evident by column temperatures. The mixture was cooled to 85°C and to the single phase mixture was added a second catalyst charge of 0.10 grams (0.002 moles) of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. After 50 minutes of reaction, methanol generation once again slowed. The mixture was cooled to 105°C and to the single phase mixture was added a third catalyst charge of 0.10 grams (0.002 moles) of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. After 50 minutes of reaction, methanol generation once again slowed. The mixture was cooled to 75°C and to the single phase mixture was added a fourth catalyst charge of 0.10 grams (0.002 moles) of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. Twenty minutes later the reaction was considered complete. At this point the yield of MEEU was 74.8%; and MEEU selectivity was 84.9%. During the reaction of the batch, the temperature at the top of the column was 25°C to 90°C and the temperature of the pot was 75°C to 110°C.

A final commercial product formulation was then prepared as follows. The mixture was cooled to 70°C and 315 grams of water was added. The flask contents were heated and the MMA-water azeotrope was removed. During the removal of excess MMA the contents were stirred and sparged. According to quantitative high-performance liquid chromatography (HPLC) the mixture contained 513 grams of product and a yellow liquid containing 48.0 weight % MEEU, 4.6 weight % HEEU, and 0.5 weight% N-(2-methacryloyloxyethyl)-N'-(methacryloyl)ethylene urea (MEMEU) and 8.4 weight % of unintentional byproducts. By Karl Fischer analysis the product contained 38.5 weight % water. A reactor efficiency ratio of 0.339 was measured by dividing pounds of MEEU produced by pounds of raw materials fed.

### Example 3

This example demonstrates the necessity of adding the catalyst at elevated temperatures, or the need to rapidly increase the mixture temperature after a catalyst charge, as well as the ability to stop and hold the reaction mixture, and to start the reaction again at a later time.

A mixture of 219.0 grams (1.68 moles) of 1-hydroxy ethylene urea (HEEU), 619.4 grams (6.19 moles) of methyl methacrylate (MMA) and 0.36 grams (0.0021 moles) of 4-hydroxy-2,2,6,6-tetramethyl piperidinyloxy, free radical (4-hydroxy TEMPO, free radical) was charged to a 1-liter 4-necked flask equipped with a temperature indicator/controller, magnetic stirrer, mixed gas (8% O₂ - 92% N₂) sparge inlet, and a 1-inch diameter-10 plate Oldershaw column fitted with a distillation head, distillate rate removal-vapor pressure temperature controller, and a graduated distillate receiver. During dehydration of the batch, the mixture was stirred, sparged with mixed gas at a rate of 70 ml/min and heated to reflux at atmospheric pressure while removing the MMA-water azeotrope. The maximum temperature atop the column was 83°C, and the maximum temperature in the flask was 100°C. At the conclusion of dehydration, the mixture was cooled to 70°C. The water content of the reaction mixture at this point was 206 ppm. To the biphasic mixture was added a first catalyst charge of 0.10 grams (0.002 moles) of lithium hydroxide monohydrate. Within five minutes of adding the catalyst, heat was applied and the temperature rose at a rate of 1.3 C/min. During reaction, the mixture was stirred, sparged with mix gas, and continuously heated to reflux at atmospheric pressure while removing the MMA-methanol of reaction azeotrope. After one hour of reaction, methanol generation dramatically slowed, as evident by column temperatures. The HEEU content was determined to be 19.2%. The mixture was cooled to 80°C and to the single phase mixture was added a second catalyst charge of 0.10 grams (0.002 moles) of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. After 45 minutes of reaction, methanol generation once again slowed. The mixture was cooled to 80°C and the HEEU determined to be 13.0%. To the single phase mixture was added a third catalyst charge of 0.10 grams (0.002 moles) of lithium hydroxide monohydrate. The mixture was allowed to sit at 80°C for 20 minutes then heat was applied and the temperature rose as previously stated. During reaction, the mixture was stirred, sparged with mix gas, and continuously heated to reflux at atmospheric pressure while removing the MMA-methanol of reaction azeotrope. After 30 minutes of reaction, methanol generation once again slowed. The mixture was cooled to 79°C and the HEEU determined to be 10.0%. To the single phase mixture was added a fourth catalyst charge of 0.10 grams (0.002 moles) of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. Twenty minutes later methanol generation once again slowed. The mixture was cooled to 90°C and the HEEU determined to be 5.8%. To the single phase mixture was added a fifth catalyst charge of 0.10 grams (0.002 moles) of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. Twenty minutes later methanol generation once again slowed. The mixture was cooled to 75°C and the HEEU determined to be 4.2%. To the single phase mixture was added a sixth catalyst charge of 0.10 grams (0.002 moles) of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. The mixture was cooled to room temperature and the HEEU content was determined to be 3.8%. Seventeen hours later (i.e., overnight), the reaction mixture was warmed to 64°C and a seventh catalyst charge of 0.10 grams (0.002 moles) of lithium hydroxide monohydrate was added. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. After 45 minutes the contents were cooled to 70°C. HEEU concentration at this point was 3.1%. During the reaction of the batch, the temperature at the top of the column was 24°C to 85°C and the temperature of the pot was 64°C to 110°C.

A final commercial product formulation was then prepared as follows. The mixture was cooled to 70°C and 420 grams of water was added. The flask contents were heated and the MMA-water azeotrope was removed. During the removal of excess MMA the contents were stirred and sparged. According to quantitative high-performance liquid chromatography (HPLC) the mixture contained 642 grams of product and a yellow liquid containing 38.5 weight % MEEU, 3.1 weight % HEEU, and 0.5 weight% N-(2-methacryloyloxyethyl)-N'-(methacryloyl)ethylene urea (MEMEU) and 11.4 weight % of unintentional byproducts. By Karl Fischer analysis the product contained 46.5 weight % water. The final yield of MEEU was 74.1% and selectivity was 81.6%. Reactor efficiency ratio, as calculated by pounds of MEEU produced divided by pounds of raw materials fed, was 0.323.

### Comparative Example 2

This example demonstrates the ineffectiveness of gradual, continuous addition of transesterification catalyst (i.e., lithium hydroxide monohydrate).

A mixture of 19.5 moles of 1-hydroxy ethylene urea (HEEU), 68.4 moles of methyl methacrylate (MMA) and 0.025 moles of 4-hydroxy-2,2,6,6-tetramethyl piperidinyloxy, free radical (4-hydroxy TEMPO, free radical) was charged to a reactor equipped with steam coils a mechanical agitator, mixed gas (8% O₂ - 92% N₂) sparge inlet, and a trayed column. For the dehydration phase, the pressure of the reactor was reduced to 260 mm Hg and the mixture was stirred, sparged with mixed gas at a rate of 100 SCFH and heated to reflux while removing the MMA-water azeotrope. The maximum temperature atop the column was 70°C. At the conclusion of dehydration, the reactor pressure was allowed to reach atmospheric pressure. The water content at this point was 510 ppm. During all phases, an inhibitor solution of MMA containing 4-hydroxy TEMPO, free radical was continuously introduced into the top of the distillation column.

Additionally, the catalyst was added gradually and continuously to the reaction mixture as follows. A catalyst solution consisting of 11 % lithium hydroxide monohydrate, 8% water, and 81% methanol was gradually and continuously added at a rate of 12 lbs/hr to the reaction mixture. During reaction, the mixture was stirred, sparged with mix gas, and continuously heated to reflux at atmospheric pressure while removing the MMA-methanol of reaction azeotrope. After three hours of reaction, the HEEU content was determined to be 18.1%. The HEEU content for the 4^{th}, 6^{th}, 7^{th} and 8^{th} hour of reaction were 16.3%, 14.9%, 13.8%, and 12.8%, respectively. At this point the rate of HEEU conversion was considered to be ineffective and, therefore, it was decided to add said catalyst as multiple separate charges, in solid form, to the reaction medium.

### Example 4

This example demonstrates how an ineffective transesterification process practiced with gradual, continuous catalyst addition (i.e., from Comparative Example 2) can be recovered by addition of multiple, separate catalyst charges.

The reaction mixture resulting from the procedure of Comparative Example 2 was cooled to 70°C and to mixture was added a first catalyst charge of (0.14 moles) of lithium hydroxide monohydrate. Within five minutes of adding the catalyst, heat was applied and the temperature initially rose at a rate of 1.3°C/min. During reaction, the mixture was stirred, sparged with mix gas, and continuously heated to reflux at atmospheric pressure while removing the MMA-methanol of reaction azeotrope. After 50 minutes of reaction, methanol generation slowed. The mixture was cooled to 74°C and the HEEU determined to be 9.2%. To the single phase mixture was added a second catalyst charge of 0.14 moles of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. After 20 minutes of reaction, methanol generation once again slowed. The mixture was cooled to 70°C and the HEEU determined to be 7.0%. To the single phase mixture was added a third catalyst charge of 0.14 moles of lithium hydroxide monohydrate. Within five minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. After 40 minutes of reaction, methanol generation once again slowed. The mixture was cooled to 74°C and the HEEU determined to be 5.5%. To the single phase mixture was added a fourth catalyst charge of 0.07 moles of lithium hydroxide monohydrate. Within five minutes of adding the catalyst, heat was applied and the temperature rose as previously stated. The mixture was stirred, sparged with mix gas, and continuously heated to reflux at atmospheric pressure for 150 minutes while removing the MMA-methanol of reaction azeotrope. The mixture was cooled to 75°C and the HEEU determined to be 4.4 %. During the reaction, the temperature at the top of the column was 55°C to 99°C and the temperature of the pot was 60°C to 106°C.

A final commercial product formulation was then prepared as follows. The mixture was cooled to 50°C and water was added, and the pressure reduced to 250 mmHg. The reactor contents were heated and the MMA-water azeotrope was removed. During the removal of excess MMA, the contents were stirred and sparged. According to quantitative high-performance liquid chromatography (HPLC) the mixture contained 49.09 weight % MEEU, 4.66 weight % HEEU, and 0.57 weight% N-(2-methacryloyloxyethyl)-N'-(methacryloyl)ethylene urea (MEMEU) and 11.44 weight % of unintentional by-products. By Karl Fischer analysis the product contained 32.81 weight % water. The final yield of MEEU was 71.3% and selectivity was 81.8%.

### Example 5

This example demonstrates the efficacy of addition of multiple, separate catalyst charges in a process wherein the transesterification catalyst is dibutyl tin oxide, in accordance with the present invention.

A reaction mixture was formed from 347 moles methyl methacrylate (MMA), an initial catalyst charge of 1.76 moles dibutyl tin oxide, and 0.12 moles of 4-hydroxy-2,2,6,6-tetramethyl piperidinyloxy, free radical (4-hydroxy TEMPO, free radical). The reaction mixture was charged to a 6000-gallon reactor equipped with steam coils a mechanical agitator, mixed gas (8% O₂- 92% N₂) sparge inlet, and a 15 tray column. For the dehydration phase, the pressure of the reactor was reduced to 260 mm Hg and the mixture was stirred, sparged with mixed gas at a rate of 400 SCFH and heated to reflux while removing the MMA-water azeotrope. The maximum temperature atop the column was 70°C. At the conclusion of dehydration, the reactor was allowed to reach atmospheric pressure and a temperature of a temperature of 55°C. The water content at this point was 900 ppm.

To the dehydrated reaction mixture was charged 118 moles of 1-hydroxy ethylene urea (HEEU). Heat was applied and the temperature rose at a rate of 1.3°C/min. During reaction, the mixture was stirred, sparged with mix gas, and continuously heated to reflux at atmospheric pressure while removing the MMA-methanol of reaction azeotrope. The HEEU content for the 10^{th}, 11^{th}, 12^{th}, and 13^{th}, hour of reaction were 16.1%, 14.0%, 11.7%, and 11.7%, respectively. At this point the rate of HEEU conversion was considered to be ineffective and, therefore, it was decided to add said catalyst in a distinct stepwise fashion to the reaction medium.

The mixture was cooled to 66°C and to the mixture was added a second catalyst charge of (0.88 moles) of dibutyl tin oxide. Within ten minutes of adding the catalyst, heat was applied and the temperature rose as previously stated. During reaction, the mixture was stirred, sparged with mix gas, and continuously heated to reflux at atmospheric pressure while removing the MMA-methanol of reaction azeotrope. After 180 minutes of reaction, methanol generation slowed. The HEEU content was determined to be 3.7%. The mixture was then cooled to 47°C. During the reaction of the batch, the temperature at the top of the column was 67°C to 99°C and the temperature of the pot was 48°C to 115°C. During all phases an inhibitor solution of MMA containing 4-hydroxy TEMPO, free radical was continuously introduced into the top of the distillation column.

A final commercial product formulation was then prepared as follows. The mixture was cooled to 50°C and the pressured reduced to 150 mm Hg, then water was added. The reactor contents were heated and the MMA-water azeotrope was removed. During the removal of excess MMA the contents were stirred and sparged. According to quantitative high-performance liquid chromatography (HPLC) the mixture contained 50.4 weight % MEEU, 3.4 weight % HEEU, and 0.6 weight% N-(2-methacryloyloxyethyl)-N'-(methacryloyl)ethylene urea (MEMEU) and 3.1 weight % of unintentional by-products. By Karl Fischer analysis the product contained 42.0 weight % water. The final yield of MEEU was 89.3% and selectivity was 98.5%.

It will be understood that the embodiments of the present invention described hereinabove are merely exemplary and that a person skilled in the art may make variations and modifications without departing from the spirit and scope of the invention. All such variations and modifications are intended to be included within the scope of the present invention.

## Claims

1. A transesterification process for the production of a (meth)acrylate ester, comprising the steps of:
(a) forming a reaction mixture, comprising:
(1) water and at least one alkyl (meth)acrylate having Formula I: 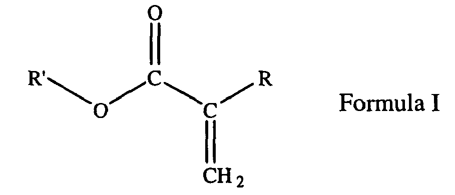 wherein R = H or CH₃; and
wherein R' = C₁-C₈ straight or branched alkyl;
(2) at least one alcohol selected from the group consisting of aliphatic linear or branched chain monoalcohols, cycloaliphatic alcohols, aromatic alcohols, functional alcohols, unsaturated alcohols, analiphatic polyols, alcohols of ethylene oxide adduct of ethylene urea, and mixtures thereof; and
(3) 10 to 10,000 ppm, based on the weight of said at least one alcohol, of at least one polymerization inhibitor;
(b) removing water from said reaction mixture by azeotropic distillation until said reaction mixture has a water content of no more than 1200 ppm based on the total weight of said reaction mixture;
(c) adding to said reaction mixture at least two charges of a transesterification catalyst, wherein each charge comprises 0.1 to 10 mole % of said transesterification catalyst, based on the total moles of said at least one alcohol,
said transesterification catalyst being selected from the group consisting of dibutyl tin oxide, reaction products of dibutyl tin oxide with components in the transesterification of various alcohols with alkyl (meth)acrylates; dibutyl tin dimethoxide, reaction products of dibutyl tin dimethoxide with components in the transesterification of various alcohols with alkyl (meth)acrylates; methanolic magnesium methylate; lithium, lithium carbonate, anhydrous alkali metal hydroxide, hydrates of alkali metal hydroxide, and mixtures thereof; and
(d) when said reaction mixture has a temperature of less than about 60°C, heating said reaction mixture to at least 60°C after addition of a first of said at least two charges of said transesterification catalyst to commence reaction of said alkyl (meth)acrylate with said at least one alcohol to form a (meth)acrylate ester having Formula II: 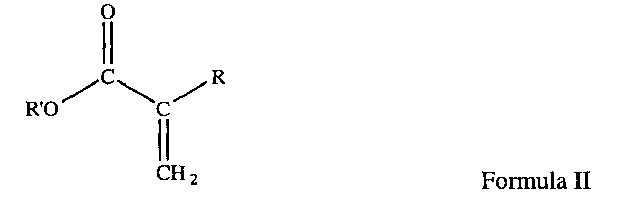 wherein R = H or CH₃, and R'O = an alkoxide of the at least one alcohol, and a product alcohol having Formula III:
R'―OH Formula III
wherein R' = C₁-C₈ straight or branched alkyl.

2. The transesterification process according to Claim 1, wherein said heating step is commenced within about 10 minutes after addition of the first of said at least two charges of said transesterification catalyst.

3. The transesterification process according to Claim 1, wherein the molar ratio of said at least one alcohol to said at least one alkyl (meth)acrylate is from 1:2 to 1:6.5.

4. The transesterification process according to Claim 1, wherein said at least one alcohol comprises an hydroxyl alkyl imidazolidin-2-one having Formula IV, as follows: 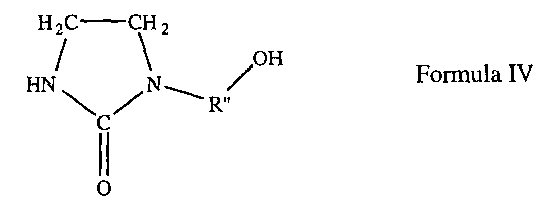 wherein R" = C₁-C₈ straight, branched or cyclic, and saturated or unsaturated, hydrocarbon, and said (meth)acrylate ester product has Formula V, as follows: 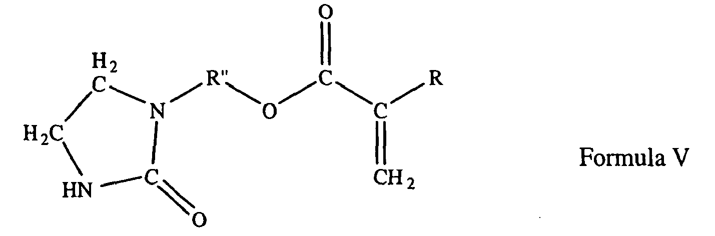 wherein R = H or CH₃, and R" = C₁-C₈ straight, branched or cyclic, and saturated or unsaturated, hydrocarbon.

5. The transesterification process according to Claim 4, wherein said hydroxyl alkyl imidazolidin-2-one is hydroxyethyl ethylene urea and said at least one alkyl (meth)acrylate comprises methyl methacrylate.

6. The transesterification process according to Claim 1, wherein said transesterification catalyst is lithium hydroxide monohydrate.

7. The transesterification process according to Claim 1, wherein said polymerization inhibitor is selected from the group consisting of oxygen, diethylhydroxylamine, p-methoxy phenol, hydroquinone, phenothiazine, 2,6-dit-butylpara-cresol, 3,5-di-t-butyl-4-hydroxyanisole, 2,5-di-t-butylhydroxyanisole, 4-hydroxy-2,2,6,6-tetramethyl piperidinyl free radical (4-hydroxy-TEMPO), 4-methacryloyloxy-2,6,6-tetrmethyl piperidinyl free radical, and 4-hydroxy-2,6,6-tetramethyl N-hydroxy piperidine and mixtures thereof.

8. The transesterification process according to Claim 1, further comprising the step of removing, by azeoptropic distillation, a mixture of said (product) alcohol of Formula III and said (reactant) alkyl (meth)acrylate of Formula I.

9. The transesterification process according to Claim 1, further comprising the step of maintaining said reaction mixture at a temperature of from 60°C to 140°C.

10. The transesterification process according to Claim 1, wherein the steps of (a) forming a reaction mixture and (b) removing water from said reaction mixture are accomplished by:
(A) forming a reaction mixture, comprising:
(i) water and at least one alkyl (meth)acrylate having Formula I: 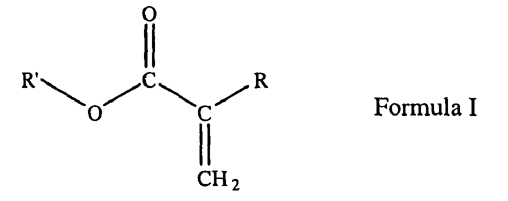 where R = H or CH₃; and
where R' = C₁-C₈ straight or branched alkyl; and
(ii) 10 to 10,000 ppm, based on the total weight of alcohol to be added, of at least one polymerization inhibitor selected from the group consisting of oxygen, diethylhydroxylamine, p-methoxy phenol, hydroquinone, phenothiazine, 2,6-dit-butylpara-cresol, 3,5-di-t-butyl-4-hydroxyanisole, 2,5-di-t-butylhydroxyanisole, 4-hydroxy-2,2,6,6-tetramethyl piperidinyl free radical (4-hydroxy-TEMPO), 4-methacryloyloxy-2,6,6-tetrmethyl piperidinyl free radical, and 4-hydroxy-2,6,6-tetramethyl N-hydroxy piperidine and mixtures thereof;
(B) removing water from said reaction mixture by azeotropic distillation until said reaction mixture has a water content of no more than 1200 ppm based on the total weight of said reaction mixture;
(C) adding to said reaction mixture at least one alcohol selected from the group consisting of aliphatic linear or branched chain monoalcohols, cycloaliphatic alcohols, aromatic alcohols, alcohols bearing other functional groups, alcohols of ethylene oxide adduct of ethylene urea, and mixtures thereof.
